# EUROPEAN PATENT APPLICATION

(11) **EP 4 091 549 A1**
(43) Date of publication of application: **23.11.2022**
(21) Application number: 20914272.8
(22) Date of filing: 09.12.2020
(51) Int. Cl.: A61B 5/346

(54) **HEARTBEAT DATA CLASSIFICATION METHOD AND DEVICE BASED ON POINT R**

(30) Priority: 17.01.2020 CN 202010057333
(71) Applicant: Shanghai Lepu CloudMed Co.,LTD, Shanghai 201612 (CN)
(72) Inventor: ZHANG, Biying, Shanghai 201612 (CN); TIAN, Liang, Shanghai 201612 (CN); CAO, Jun, Shanghai 201612 (CN)
(74) Representative: Glück Kritzenberger Patentanwälte PartGmbB
(86) International application number: PCT/CN2020/134748
(87) International publication number: WO 2021/143400

(57) **Abstract**

Disclosed are a heartbeat data classification method and device based on a point R. The method comprises: (1) obtaining one-dimensional electrocardiogram data of which a time length is a preset fragment time threshold to generate an electrocardiogram data fragment, and calling a target detection algorithm to perform feature recognition of heartbeat signal data on the electrocardiogram data fragment to generate a sequence of first bounding box, wherein the sequence of first bounding box comprises a plurality of first bounding boxes; (2) performing absolute value conversion on all the first bounding boxes in the sequence of first bounding box to generate a sequence of second bounding box, and performing non-maximum suppression on the sequence of second bounding box, wherein the sequence of second bounding box comprises a plurality of second bounding boxes, each second bounding box comprises a group of probability for point R heartbeat classification , and the group of heartbeat classification probability comprises at least one class of parameters for heartbeat classification probability ; (3) performing valid parameter and invalid parameter marking on all the parameters for heartbeat classification probability of the group of heartbeat classification probability of all the second bounding boxes in the sequence of second bounding box; and (4) performing point R position information and valid parameter extraction on all the second bounding boxes in the sequence of second bounding box in chronological order to generate a sequence for point R position and heartbeat data classification information.

## Description

The application claims priority to Chinese Patent Application No. 202010057333.3, entitled "HEARTBEAT DATA CLASSIFICATION METHOD AND DEVICE BASED ON POINT R", filed with the China National Intellectual Property Administration on January 17, 2020.

### BACKGROUND OF THE INVENTION

### 1. Technical Field

The invention relates to the technical field of electrocardiogram (ECG ) signal processing, in particular to a heartbeat data classification method and device based on a point R.

### 2. Description of Related Art

Electrocardiogram (ECG) data is a set of electric signal data relating to the heart cardiac cycle, and ECG analysis is feature analysis performed on the collected electrocardiogram data. A common electrocardiogram data waveform has five feature points, which are a point P, a point Q, a point R, a point S and a point T respectively. Electrocardiogram data is analyzed by recognizing and extracting feature point information first and then selecting a classification method to perform classification. Existing methods for recognizing the feature points of electrocardiogram data have recognition requirements for all these five points. In actual operation, the point P and the point T are likely to be interfered by noise signals and are quite likely to be eliminated mistakenly during signal filtering and noise cancellation, which may indirectly result in the loss of corresponding heartbeat signals. In this case, an output analysis report may have the problems of feature omissions and deviations.

### BRIEF SUMMARY OF THE INVENTION

The object of the invention is to overcome the defects of the prior art by providing a heartbeat data classification method and device based on a point R. First, the strongest signal, the point R signal, of the five points is used as a heartbeat signal feature point, so that heartbeat signal data is reserved to the maximum extent to be used as an analysis source, and the problem of heartbeat data loss of conventional methods is solved; and heartbeat signals with the point R as the main feature point are further subjected to heartbeat classification to be output.

To fulfill the above object, in a first aspect, the embodiments of the invention provide a heartbeat data classification method based on a point R, comprising:

Obtaining one-dimensional electrocardiogram data of which a time length is a preset fragment time threshold to generate an electrocardiogram data fragment, and calling a target detection algorithm to perform recognition of heartbeat signal data feature on the electrocardiogram data fragment to generate a sequence of first bounding box, wherein the sequence of first bounding box comprises a plurality of first bounding boxes;

Performing absolute value conversion on all the first bounding boxes in the sequence of first bounding box to generate a sequence of second bounding box, and performing non-maximum suppression on the sequence of second bounding box, wherein the sequence of second bounding box comprises a plurality of second bounding boxes, each second bounding box comprises a group of point R heartbeat classification probability, and the group of heartbeat classification probability comprises at least one class of parameters for heartbeat classification probability;

Performing valid parameter and invalid parameter marking on all parameters of the heartbeat classification probability in the group of heartbeat classification probability of all the second bounding boxes in the sequence of second bounding box; and

Performing point R position information and valid parameter extraction on all the second bounding boxes in the sequence of second bounding box in chronological order to generate a sequence for point R position and heartbeat data classification information.

Preferably, the step of obtaining one-dimensional electrocardiogram data of which a time length is a preset fragment time threshold to generate an electrocardiogram data fragment and calling a target detection algorithm to perform heartbeat signal data feature recognition on the electrocardiogram data fragment to generate a sequence of first bounding box comprises:
Obtaining the one-dimensional electrocardiogram data of which the time length is the preset fragment time threshold to generate the electrocardiogram data fragment; and
Calling the target detection algorithm to perform average grid division on the electrocardiogram data fragment with a preset grid time threshold as a grid division step to generate a fragment grid group, performing recognition of heartbeat signal data feature on fragment grids to generate the plurality of first bounding boxes, and collecting all the first bounding boxes generated by all the fragment grids in chronological order of the grids to generate the sequence the first bounding box, wherein the fragment grid group comprises a plurality of fragment grids, each first bounding box comprises a group of first heartbeat signal probability, point R relative time data, a QRS normalized time width and a first heartbeat classification probability, and the sequence of first bounding box comprises the plurality of first bounding boxes.

Preferably, the step of performing absolute value conversion on all the first bounding boxes in the sequence of first bounding box to generate a sequence of second bounding box and performing non-maximum suppression on the sequence of second bounding box comprises:
Step 31, initializing the sequence of second bounding box to be null; initializing the value of a first index to 1, and initializing a first total number to the total number of the first bounding boxes in the sequence of first bounding box;
Step 32, setting a second bounding box corresponding to the first index; initializing a second heartbeat signal probability of the second bounding box corresponding to the first index to be null, initializing point R absolute time data of the second bounding box corresponding to the first index to be null, initializing a QRS absolute time width of the second bounding box corresponding to the first index to be null, and initializing the group of heartbeat classification probability for the second bounding box corresponding to the first index to be null;
Step 33, setting the probability of second heartbeat signal of the second bounding box corresponding to the first index to the probability of first heartbeat signal of the first bounding box, corresponding to the first index, in the sequence of first bounding box, and setting the group of heartbeat classification probability of the second bounding box corresponding to the first index to the group of first heartbeat classification probability of the first bounding box, corresponding to the first index, in the sequence of first bounding box;
Step 34, extracting the point R relative time data of the first bounding box, corresponding to the first index, in the sequence of first bounding box to generate an intra-grid time migration data, adding 1 to a result obtained by rounding a quotient of a difference, obtained by subtracting 1 from the first index, and a preset threshold of the number of bounding boxes in unit grids to generate a grid index of the bounding box, generating grid starting time data according to a product of a difference, obtained by subtracting 1 from the grid index of the bounding box, and the preset grid time threshold, and setting the point R absolute time data of the second bounding box corresponding to the first index to the sum of the grid starting time data and the intra-grid time migration data;
Step 35, extracting the QRS normalized time width of the first bounding box, corresponding to the first index, in the sequence of first bounding box to generate a time width normalized value, and setting the QRS absolute time width of the second bounding box, corresponding to the first index to a product of a square of the time width normalized value and the preset fragment time threshold;
Step 36, adding the second bounding box corresponding to the first index into the sequence of second bounding box as a bounding box object;
Step 37, adding 1 to the value of the first index;
Step 38, determining whether the first index is greater than the first total number; if the first index is greater than the first total number, performing Step 39; or, if the first index is smaller than or equal to the first total number, performing Step 32;
Step 39, sequentially performing query of heartbeat signal probability on all the second bounding boxes in the sequence of second bounding box; and when the probability of second heartbeat signal of a currently queried second bounding box is out of a preset threshold range of heartbeat probability, deleting the currently queried second bounding box from the sequence of second bounding box; and
Step 40, comparing every two of all the second bounding boxes in the sequence of second bounding box; when a time overlap proportion of the two compared second bounding boxes is out of a preset bounding box overlap proportion threshold range, deleting the second bounding box with a probability of smaller second heartbeat signal in the two compared second bounding boxes from the second bounding box sequence.

Preferably, the step of performing valid parameter and invalid parameter marking on all the heartbeat classification probability parameters in the heartbeat classification probability group of all the second bounding boxes in the second bounding box sequence comprises:
sequentially querying all the second bounding boxes in the sequence of second bounding box, making a maximum parameter of heartbeat classification probability in the group of heartbeat classification probability a currently queried second bounding box as a valid parameter, and marking the other parameters of heartbeat classification probability smaller than the maximum parameter of heart heartbeat classification probability in the group of heartbeat classification probability of the currently queried second bounding box as invalid parameters.

Preferably, the step of performing point R position information and valid parameter extraction on all the sequence of second bounding boxes in the second bounding box in chronological order to generate a sequence of point R position and heartbeat data classification information specifically comprises:
Step 51, chronologically reordering all the sequence of second bounding boxes in the second bounding box according to the point R absolute time data;
Step 52, initializing the sequence of point R position and heartbeat data classification information to be null; initializing a first temporary sequence to be null; initializing the value of a second index to 1, and initializing a second total number to the total number of the second bounding boxes in the sequence of second bounding box;
Step 53, setting information for second index point R position and heartbeat data classification; initializing point R position information of the second index point R position and heartbeat data classification information to be null, and initializing QRS width information of the second index point R position and heartbeat data classification information to be null; initializing a group of valid heartbeat classification probability of the second index point R position and heartbeat data classification information to be null;
Step 54, calculating the total number of parameters of heartbeat classification probability, marked as valid parameters, in the group of heartbeat classification probability of the second bounding box, corresponding to the second index, in the sequence of second bounding box to generate the total number of valid classification parameters;
Step 55, determining whether the total number of valid parameters of classification is equal to 0; if the total number of valid parameters of classification is greater than 0, performing Step 56; or, if the total number of valid classification parameters is equal to 0, performing Step 58;
Step 56, setting the point R position information of the point R position and heartbeat data classification information with the second index to the point R absolute time of the second bounding box, corresponding to the second index, in the sequence of second bounding box; setting the QRS width information of the point R position and heartbeat data classification information with the second index to the QRS absolute time width of the second bounding box, corresponding to the second index, in the sequence of second bounding box; extracting all parameters of probability for heartbeat classification , marked as valid parameters, in the group of heartbeat classification probability of the second bounding box, corresponding to the second index, in the sequence of second bounding box, and sequentially adding parameters of heartbeat classification probability into the group of valid heartbeat classification probability of the point R position and heartbeat data classification information with the second index;
Step 57, adding the point R position and heartbeat data classification information with the second index into the first temporary sequence;
Step 58, adding 1 to the value of the second index;
Step 59, determining whether the second index is greater than the second total number; if the second index is greater than the second total number, performing Step 60; or, if the second index is smaller than or equal to the second total number, performing Step 53; and
Step 60, extracting all point R position and heartbeat data classification information in the temporary sequence, and sequentially adding the point R position and heartbeat data classification information into the sequence of point R position and heartbeat data classification information.

According to the heartbeat data classification method based on a point R provided in the first aspect of the embodiments of the invention, a target detection algorithm is used to perform target recognition on a heartbeat signal feature point (point R) in an electrocardiogram data fragment of a fixed length, and a sequence of bounding box containing point R recognition information is output. In the embodiments of the invention, after a recognition output result is obtained, absolute value conversion and non-maximum suppression are further performed on the bounding box sequence to obtain an optimized bounding box sequence; then, validity marking is performed on the heartbeat classification parameter in the optimized bounding box; and finally, position information for the heartbeat signal feature point (point R) and valid heartbeat classification information are extracted from the marked sequence of box to generate a point R position and sequence for heartbeat data classification information. By adoption of the method of the invention, the point R in heartbeat signals is used as the heartbeat signal feature point, so that heartbeat signal data can be reserved to the maximum extent, and output settings for various analysis report can be made based on the point R position and the sequence for heartbeat data classification information.

In a second aspect, the embodiments of the invention provide a device, comprising a memory and processor, wherein the memory is used to stores a program, and the processor is used to implement the method in the first aspect and various implementations of the first aspect.

In a third aspect, the embodiments of the invention provide a computer program product including an instruction. When the computer program product runs on a computer, the computer is enabled to implement the method in the first aspect and various implementations of the first aspect.

In a fourth aspect, the embodiments of the invention provide a computer-readable storage medium which stores a computer program, and when the computer program is executed by a processor, the method in the first aspect and various implementations of the first aspect is implemented.

### BRIEF DESCRIPTION OF THE SEVERAL VIEWS OF THE DRAWINGS

FIG. 1 is a schematic diagram of a heartbeat data classification method based on a point R according to a first embodiment of the invention;
FIG. 2 is a schematic diagram of a heartbeat signal according to one embodiment of the invention;
FIG. 3 is a schematic diagram of performing valid parameter and invalid parameter marking on all heartbeat classification probability parameters in a heartbeat classification probability group according to a second embodiment of the invention;
FIG. 4 is a structural diagram of a heartbeat data classification device based on a point R according to a third embodiment of the invention.

### DETAILED DESCRIPTION OF THE INVENTION

To make the purposes, technical solutions, and advantages of the invention clearer, the invention will be described in further detail below in conjunction with the accompanying drawings. Obviously, the embodiments in the following description are merely illustrative ones, and are not all possible ones of the invention. All other embodiments obtained by those ordinarily skilled in the art based on the following ones without creative labor should also fall within the protection scope of the invention.

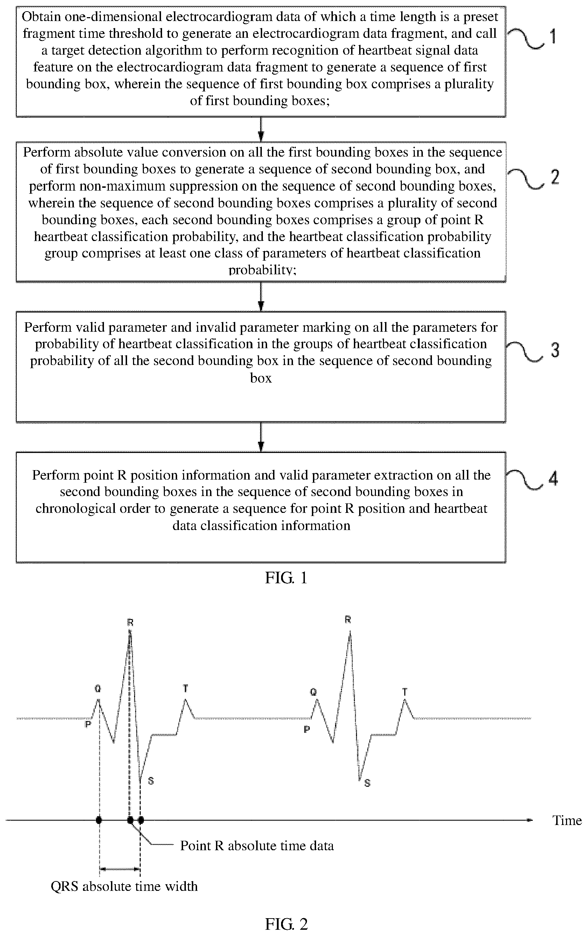

As shown in FIG. 1 which is schematic diagram of a heartbeat data classification method based on a point R according to a first embodiment of the invention, the method mainly comprises the following steps:
Step 1, one-dimensional electrocardiogram data of which a time length is a preset fragment time threshold is obtained to generate an electrocardiogram data fragment, and a target detection algorithm is called to perform recognizing the feature of the heartbeat signal data on the electrocardiogram data fragment to generate a sequence of first bounding box;
Wherein, the first bounding box sequence comprises a plurality of first bounding boxes;
Step 1 comprises: Step 11, the one-dimensional electrocardiogram data of which the time length is the preset fragment time threshold is obtained to generate the electrocardiogram data fragment;
Here, the one-dimensional electrocardiogram data mentioned in this specification is generated by extracting time information for heartbeat signal from electrocardiogram lead data and is a piece of data information for heartbeat signal of which the length is a preset fragment time threshold. As shown in FIG. 2 which is a schematic diagram of a heartbeat signal according to one embodiment of the invention, the piece of data information for heartbeat signal consists of multiple pieces of heartbeat signal data, and each piece of heartbeat signal data comprises five feature points P, Q, R, S and T; it can be seen from FIG. 2, the point R in the five points has the highest peak, and compared with the point P and the point T, the point R has the best anti-interference capacity. So, compared with traditional methods for recognizing heartbeat signals through these five points, the method for recognizing heartbeat signals through the point R can improve the recognition precision of valid signals. In the method of the invention, the target detection algorithm is called to recognize a set of point R absolute time data, a QRS absolute time width and a heartbeat classification probability group related to the point R, then marking validity is performed on the heartbeat classification probability group, and finally, point R information with a valid heartbeat classification probability group is extracted to form a sequence for point R position and heartbeat data classification information;
Step 12, the target detection algorithm is called to perform average grid division on the electrocardiogram data fragment with a preset time threshold for grid as a grid-division-step to generate a group of grids, recognizing feature for heartbeat signal data is performed on grids in ECG fragment to generate a plurality of first bounding boxes, and all the first bounding boxes generated by all the grids are collected in chronological order of the grids to generate a sequence of first bounding box;

Wherein, the group of fragment grids comprises a plurality of fragment grids; each first bounding box comprises a first heartbeat signal probability, point R relative time data, a QRS normalized time width and a first heartbeat classification probability group; and the sequence for first bounding box comprises a plurality of first bounding boxes.

Here, the target detection algorithm is briefly introduced: the target detection algorithm involved in this embodiment of the invention uses a heartbeat signal prediction network model which is trained based on the CNN (Convolutional Neural Network) principle. The prediction network model averagely divides the electrocardiogram data fragment with a fixed time length into a plurality of time grids, prediction of point R is performed on electrocardiogram data in each time grid, and finally, a plurality of bounding boxes for prediction point R are predicted in each time grid. An ideal bounding box for prediction includes one heartbeat signal at most, that is, bounding box includes one point R at most. The bounding box, as a data object output by a prediction network, not only possesses time width information of its own, but also comprises a heartbeat signal probability, that is the possible probability if the electrocardiogram data in a current time period for bounding box is a heartbeat signal, point R relative time data , that is the relative displacement of a point R signal in the current time period for bounding box with respect to a starting time of time grid, a QRS normalized time width, that is the normalized value of a wave R signal's time width in the current bounding box time period for bounding box with respect to the time length of the electrocardiogram data fragment, the wave R signal time width is represented by a QRS time width, and a heartbeat classification probability group , that is the possible probability of electrocardiogram data in the current time period for bounding box with respect to multiple heartbeat classes; in addition, the data input length of the prediction network model is restrained by software and hardware resource in specific implementation; if the time length of the one-dimensional electrocardiogram data exceeds the input length of the prediction network model, fragmentation needs to be performed on the one-dimensional electrocardiogram data according to the following principle: a preset time threshold of fragment is specified according to the maximum input length of the prediction network model, one-dimensional electrocardiogram data of a fixed length is obtained according to the time threshold of fragment to generate an electrocardiogram data fragment, and then the electrocardiogram data fragment is input to the prediction network model to perform prediction for point R information.

Here, the target detection algorithm averagely divides the electrocardiogram data fragment of which the length is the preset time threshold for fragment into a plurality of fragment grids by means of the prediction network model, wherein the time length of each fragment grid is equal to a preset time threshold for grid; after grid division is completed by the prediction network model, point R prediction is performed on electrocardiogram data in each fragment grid by calculation, and a plurality of point R prediction bounding box , that is first bounding box, are predicted eventually, wherein as mentioned above, each prediction bounding box i.e. first bounding box, comprises at least four data items: the first heartbeat signal probability, the point R relative time data, the QRS normalized time width and the first heartbeat classification probability group; and finally, the prediction bounding box of all the grids are collected by the target detection algorithm to generate the sequence for first bounding boxes;

Assume the electrocardiogram data fragment is divided into Y grids by the target detection algorithm in actual implementation and each grid outputs the amount of Z first bounding boxes, the sequence of first bounding boxes bounding box comprises the amount of Y^{∗}Z first bounding boxes.
Step 2, absolute value conversion is performed on all the first bounding boxes in the sequence of first bounding boxes to generate a sequence of second bounding boxes, and non-maximum suppression is performed on the sequence of second bounding boxes;
Wherein, the sequence of second bounding boxes comprises a plurality of second bounding boxes, each second bounding box comprises a group of heartbeat classification probability of the point R , and the group of heartbeat classification probability comprises at least one class of heartbeat classification probability parameters;
Step 2 includes: Step 21, the sequence of second bounding box is initialized to be null; the value of a first index is initialized to 1, and a first total number is initialized to be the total number of the first bounding box in the sequence of first bounding boxes;
Step 22, a second bounding box corresponding to the first index is set; a second heartbeat signal probability of the second bounding box corresponding to the first index is initialized to be null, absolute time data of point R of the first second bounding box corresponding to the first index is initialized to be null, a QRS absolute time width of the second bounding box corresponding to the first index is initialized to be null, and the group of heartbeat classification probability of the second bounding box corresponding to the first index is initialized to be null;
Step 23, the second heartbeat signal probability of the second bounding box corresponding to the first index is set to the first heartbeat signal probability of the first bounding box corresponding to the first index, in the sequence of first bounding box; the group of heartbeat classification probability of the second bounding box corresponding to the first index is set as the group of first heartbeat classification probability of the first bounding box, corresponding to the first index, in the sequence of first bounding boxes;
Step 24, the relative time of point R of the first bounding box, corresponding to the first index, in the sequence of first bounding box is extracted to generate time migration in a grid, 1 is added to a result obtained by rounding a quotient of a difference, obtained by subtracting 1 from the first index, and a preset threshold of the number of bounding boxes in unit grids to generate an index of a grid corresponding to the bounding box, start time of a grid is generated according to a product of a different, obtained by subtracting 1 from the index of the grid corresponding to bounding boxes, and a preset time threshold of a grid ,and the absolute time of point R of the second bounding box corresponding to the first index is set to the sum of the start time of a grid and the time migration in a grid;
Herein, the start time of a grid = (the index of the grid corresponding to the bounding box-1)^{∗}the preset time threshold of a grid, and the absolute time of point R = the relative time of point R + start time of the grid;
Herein, due to the fact that one grid may output multiple bounding boxes of which the specific number depends on the preset threshold of the number of bounding boxes in one unit grid and the sequence of first bounding boxes is formed by the bounding boxes sequentially extracted from each grid in chronological order of the grids, when the relative time of point R of each bounding box is converted, the index of a grid to which the current bounding box belongs, namely the index of the grid of the bounding box, needs to be determined, and here, the index of the grid of the bounding box = |(first index -1)/the preset threshold of the number of bounding boxes in unit grids |+1. For example, assume an ECG electrocardiogram data fragment comprises Y grid fragments and each fragment of grid comprises Z first bounding boxes, wherein, Z is the preset threshold of the number of bounding b boxes in one unit grids, the sequence for first bounding box comprises Y^{∗}Z first bounding boxes, and every Z successive first bounding boxes belong to one grid fragment. For another example, assume an electrocardiogram data fragment comprises three grids and each fragment grid comprises two first bounding boxes, the sequence of first bounding box comprises 3^{∗}2=6 first bounding boxes {bounding box₁₁, bounding box₁₂, bounding box₂₁, bounding box₂₂, bounding box₃₁, bounding box32}; when the first index is 1 and 2, the first bounding boxes corresponding to the first index are bounding box₁₁ and bounding box₁₂ respectively, and the grid index of the bounding boxes meets |(1-1)/2|+1=1 and |(2-1)/2|+1=1, which indicates that a grid to which the bounding box₁₁ and bounding box₁₂ belong is the first grid; when the first index is 3 and 4, a grid to which the bounding box₁₃ and bounding box₁₄ belong is the second grid; and when the first index is 5 and 6, a grid to which the bounding box₁₅ and bounding box₁₆ belong is the third grid;
Step 25, the QRS normalized time width of the first bounding box, corresponding to the first index, in the sequence of first bounding box is extracted to generate a time width normalized value, and the QRS absolute time width of the second bounding box corresponding to the first index is set to a product of the square of the time width normalized value and the preset threshold of fragment time;
Step 26, the second bounding box corresponding to the first index is added into the sequence of second bounding box as a bounding box object;
Step 27, 1 is added to the first index;
Step 28, whether the first index is greater than the first total number is determined; if the first index is greater than the first total number, Step 29 is performed; or, if the first index is smaller than or equal to the first total number, Step 22 is performed;
Step 29, heartbeat signal probability is queried sequentially on all the second bounding boxes in the sequence of second bounding box; when the second heartbeat signal probability of a currently queried second bounding box is out of a preset threshold range of heartbeat signal probability, the currently queried second bounding box is deleted from the sequence of second bounding box;

Step 30, every two of all the second bounding boxes in the sequence of second bounding box are compared; and when a time overlap proportion of two compared second bounding boxes is out of a preset threshold range for bounding box's overlap proportion, the second bounding box with a smaller second heartbeat signal probability in the two compared bounding boxes is deleted from the sequence of second bounding box.

Here, Step 2 is explained in detail through Steps 21-30. In Step 2, considering the huge floating-point calculation of convolution, in order to improve the calculation efficiency, the time value of the point R and the QRS time width are calculated relatively when the target detection algorithm predicts the bounding boxes by means of the prediction network model. So, in order to extract the absolute position information of the point R, conversion of absolute value needs to be performed on the point R relative time and the QRS normalized time width of the first bounding boxes in the sequence of first bounding box, and bounding boxes obtained after conversion are defined as second bounding boxes. Assume the first sequence of bounding box comprises Y^{∗}Z first bounding boxes, the sequence of second bounding box comprises Y^{∗}Z second bounding boxes. The data structure of the second bounding boxes and the data structure of the first bounding boxes are briefly introduced in the following table.

**Table 1**

| First bounding box | | Second bounding box | | Explanation |
|---|---|---|---|---|
| Parameters | Parameter description | Parameters | Parameter description | |
| First heartbeat signal probability | Probability that electrocardiogram data in the current bounding box time period is a heartbeat signal | Second heartbeat signal probability | Probability that electrocardiogram data in the current bounding box time period is a heartbeat signal | Second heartbeat signal probability = first heartbeat signal probability |
| Point R relative time | Relative displacement of a point R signal in the current bounding box time period with respect to a grid's start time | Point R absolute time data | Relative displacement of the current bounding box time period with respect to the start time of an electrocardiogram data fragment | Point R relative time data = start time of the grid to which point R belongs + point R's relative time, wherein start time of the grid to which point R belongs = preset time threshold of the grid X (grid index of the bounding box-1) |
| QRS normalized time width | Normalized value of a wave R signal time width in the current bounding box's time period with respect to an electrocardiogram data fragment | QRS absolute time width | Absolute width of a wave R signal width time in the current bounding box's time period | QRS absolute time width = QRS normalized time width 2 ^{∗} preset time threshold of fragment |
| Group of probability of first heartbeat classificati on | Possible probability of electrocardiogram data in the current bounding box's time period with respect to multiple heartbeat classes | group of probability of heartbeat classification | Possible probability of electrocardiogram data in the current bounding box's time period with respect to multiple heartbeat classes | probability group of heartbeat classification = probability group of first heartbeat classification |

The sequence of second bounding box is generated after absolute value conversion is performed on the sequence of first bounding box, and then non-maximum suppression is performed on the sequence of second bounding box in Step 2. This process comprises two optimization steps: 1, optimization of heartbeat signal probability is performed on the sequence of second bounding box, that is, second bounding boxes of which the probability of second heartbeat signal is not within a preset threshold of heartbeat signal probability are determined as optimization objects and are deleted from the sequence of second bounding box; 2, overlap optimization is performed on the sequence of second bounding box, that is, every two of the second bounding boxes in the sequence of second bounding box are compared; if the two compared second bounding boxes overlap in time, an overlap proportion is calculated; and when the time overlap proportion is out of a preset proportion range for bounding box overlap , the second bounding box with a smaller second heartbeat signal probability in the two compared second bounding boxes is determined as an optimization object and is deleted from the sequence of second bounding box; if the sequence of second bounding box comprises N second bounding boxes after these two optimization steps are completed, N≤Y^{∗}Z.

Step 3, marking valid parameter and invalid parameter is performed on all the parameters for probability of heartbeat classification in the groups of heartbeat classification probability of all the second bounding boxes in the sequence of second bounding box;

Step 3 includes: all the second bounding boxes in the sequence of second bounding box are queried in turns; a parameter of probability for heartbeat classification with maximum value in the group of heartbeat classification probability in a currently queried second bounding box is marked as a valid parameter, and the other parameters of probability for heartbeat classification which have smaller values than the maximum in the group of probability for heartbeat classification of the currently queried second bounding box are marked as invalid parameters.

Here, in Step 3, marking heartbeat types is further performed on all the second bounding boxes in the sequence of second bounding box based on the groups of probability for heartbeat classification. For example, the group of probability for heartbeat classification of each second bounding box includes four parameters of probability for heartbeat classification: parameters of probability for heartbeat classification A(B/C/D); and when the second bounding boxes in the sequence of second bounding box are queried, the parameters of probability for heartbeat classification A(B/C/D) are compared, and a parameter with maximum value in these four parameters is marked as a valid parameter, and the other three parameters are marked as invalid parameters.

Step 4, Extracting position information and valid parameter of point R is performed on all the second bounding boxes in the sequence of second bounding box in chronological order, to generate a sequence for point R position and heartbeat data classification information;

Step 4 includes: Step 41, all the second bounding boxes in the sequence of second bounding box are reordered chronologically according to the absolute time of point R;
Step 42, the sequence of point R position and heartbeat data classification information is initialized to be null; a first temporary sequence is initialized to be null; the value of a second index is initialized to 1, and a second total number is initialized to be the total number of the second bounding b boxes in the sequence of second bounding box;
Step 43, point R position and heartbeat data classification information with second index is set; point R position information of the point R position and heartbeat data classification information with the second index is initialized to be null, QRS width information of the point R position and heartbeat data classification information with the second index is initialized to be null; and a group of probability of valid heartbeat classification of point R position and heartbeat data classification information with the second index is initialized to be null;
Step 44, the total number of parameters of probability for heartbeat classification, marked as valid parameters, in the group of probability for heartbeat classification of the second bounding box, corresponding to the second index, in the sequence for second bounding box is calculated to generate a total number of valid classification parameters;
Step 45, whether the total number of valid parameters of classification is equal to 0 is determined; if the total number of valid parameters of classification is greater than 0, Step 46 is performed; or, if the total number of valid parameters of classification is equal to 0, Step 48 is performed;
Step 46, the point R position information of the point R position and heartbeat data classification information with the second index is set to the point R's absolute time of the second bounding box, corresponding to the second index, in the sequence for second bounding box; the QRS width information of the point R position and heartbeat data classification information with the second index is set as the QRS absolute time width of the second bounding box, corresponding to the second index, in the sequence of second bounding box; all parameters of probability for heartbeat classification, marked as valid parameters, in the group of heartbeat classification probability of the second bounding box, corresponding to the second index, in the sequence for second bounding box are extracted, and parameters for heartbeat classification probability are sequentially added into the group of valid heartbeat classification probability of the point R position and heartbeat data classification information with the second index;
Step 47, the point R position and heartbeat data classification information with the second index is added into the first temporary sequence;
Step 48, 1 is added to the second index;
Step 49, whether the second index is greater than the second total number is determined; if the second index is greater than the second total number, Step 50 is performed; or, if the second index is smaller than or equal to the second total number, Step 43 is performed;

Step 50, all point R position and heartbeat data classification information in the first temporary sequence is extracted, and point R position and heartbeat classification information is sequentially added into the sequence for point R position and heartbeat data classification information.

Here, Steps 41-50 provide a detailed explanation of the generation process of the sequence for point R position and heartbeat data classification information. Due to the fact that the second bounding boxes in the second bounding box sequence may be no longer in chronological order after absolute value conversion and two times of optimization are performed in the sequence of second bounding box in Step 2, all the second bounding boxes (data items) in the sequence of second bounding box are reordered chronologically according to the absolute time of point R of the second bounding boxes first; then, the second bounding box including the absolute time of point R , the parameters for QRS absolute time width and the groups of heartbeat classification probability of the second bounding boxes in the reordered sequence for second bounding box are extracted in turn; next, whether valid parameters of heartbeat classification exist in the groups of heartbeat classification probability are determined by retrieval; if so, valid parameters for heartbeat classification probability in the group of heartbeat classification probability of a current second bounding box are extracted to generate a valid group of heartbeat classification probability, and the point R absolute time, the QRS absolute time width and the valid group of heartbeat classification probability of the current second bounding box are added into the sequence of point R position and heartbeat classification information.

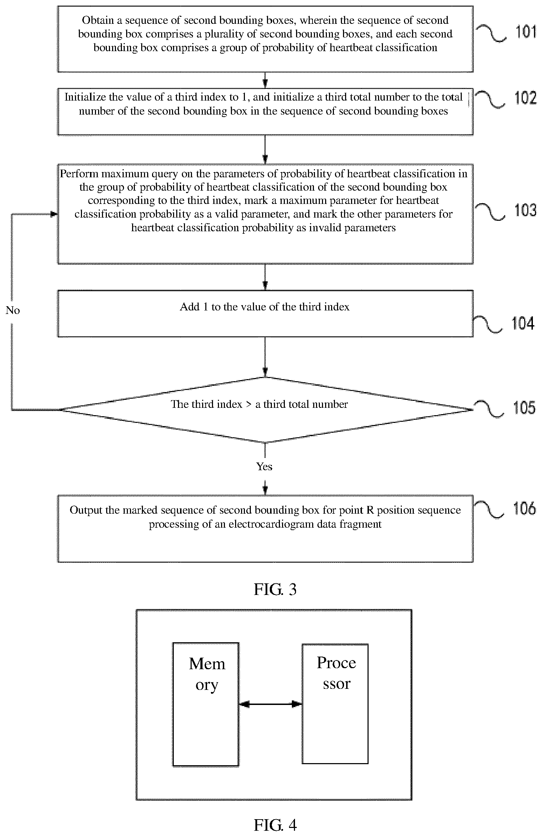

FIG. 3 is a schematic diagram of performing valid parameter and invalid parameter marking on all parameters for heartbeat classification probability in the group of heartbeat classification probability according to a second embodiment of the invention. The second embodiment is used to explain the step of performing valid parameter and invalid parameter marking on all parameters for heartbeat classification probability in the group of heartbeat classification probability in further detail. The method mainly comprises the following steps:
Step 101, a sequence of second bounding box is obtained;

Wherein, the sequence of second bounding box comprises a plurality of second bounding boxes, each second bounding box comprises a group of heartbeat classification probability, and the group of heartbeat classification probability comprises at least one class of heartbeat classification probability parameters.

Assume the sequence of second bounding box comprises four second bounding boxes, that is bounding box 1, bounding box 2, bounding box 3 and bounding box 4, and the group of heartbeat classification probability of each second bounding box comprises four classes of heartbeat classification probability parameters, as shown in the following table:

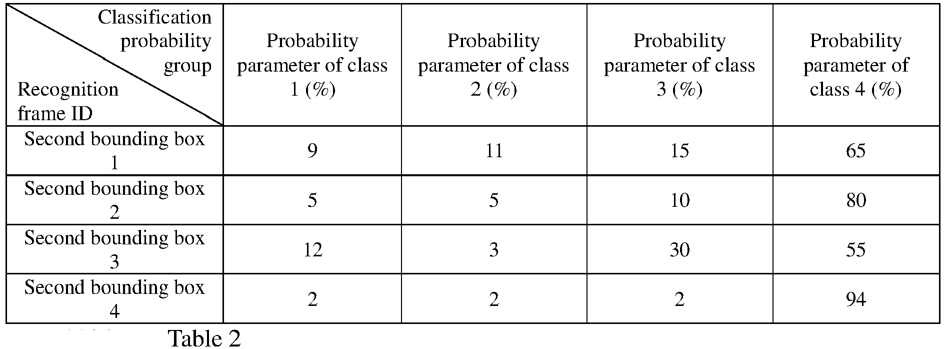

Step 102, the value of a third index is initialized to 1, and a third total number is initialized to the total number of the second bounding boxes in the sequence of second bounding box.

Step 103, querying maximum is performed on the parameters of heartbeat classification probability in the group of heartbeat classification probability of the second bounding box corresponding to the third index, a maximum parameter of heartbeat classification probability is marked as a valid parameter, and the other parameters of heartbeat classification probability are marked as invalid parameters.

Here, Step 103 is a specific process for marking the parameters of heartbeat classification probability in a case where the third index is 1. As shown in Table 2, the second bounding box 1 comprises four parameters for heartbeat classification probability, wherein the parameter of class probability 1 is 9%, the parameter of class probability 2 is 11%, the parameter of class probability 3 is 15%, and the parameter of class probability 4 is 65%, as shown in Table 2; the parameter of class probability 4 in the group of heartbeat classification probability of the second bounding box1 has the maximum value, so the parameter of class probability 4 is marked as a valid parameter, and the other parameters are marked as invalid parameters. Marking results of the second bounding box 1 are shown in the following table.

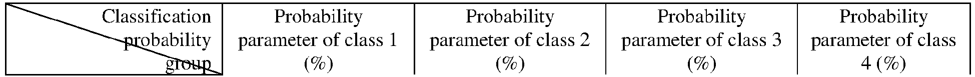

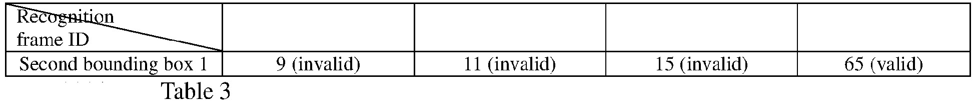

Step 104, 1 is added to the value of the third index.

Step 105, whether the third index is greater than the third total number is determined; if the third index is greater than the third total number, Step 106 is performed; or, if the third index is smaller than or equal to the third total number, Step 103 is performed.

Step 106, the marked sequence for second bounding box is output into process of the sequence of point R position of the electrocardiogram data fragment.

Here, through marking operations in Steps 101-106, marking results of the sequence of second bounding box in Table 2 are shown in the following table:

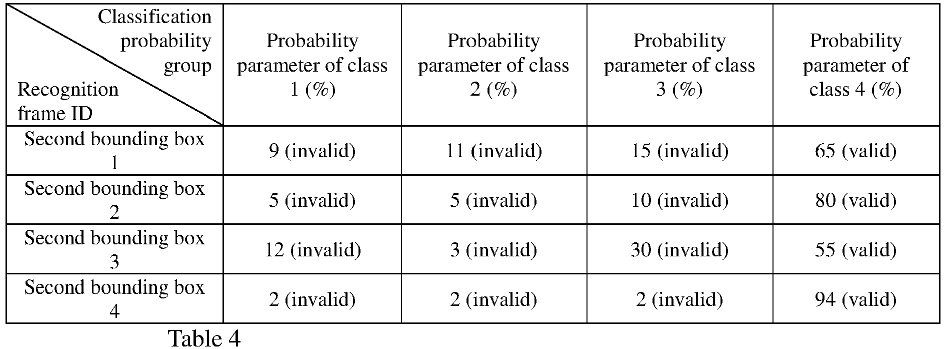

FIG. 4 is a schematic diagram of a heartbeat data classification device based on a point R according to a third embodiment of the invention. The device comprises: a processor and a memory. The memory is connected to the processor through a bus. The memory may be a non-volatile memory such as a hard disk drive or a flash memory. A software program and a device drive program are stored in the memory. The software program can execute various functions of the method provided by the embodiments of the invention. The device drive program may be a network and interface drive program. The processor is used to execute the software program, and when the software program is executed, the method provided by the embodiments of the invention is implemented.

It should be noted that one embodiment of the invention further provides a computer-readable storage medium. The computer-readable storage medium stores a computer program, and when the computer program is executed by a processor, the method provided by the embodiments of the invention is implemented.

One embodiment of the invention further provides a computer program product including an instruction. When the computer program product runs on a computer, a processor is enabled to implement the method mentioned above.

According to the heartbeat data classification method and device based on a point R provided by the embodiments of the invention, a target detection algorithm is used to perform target recognition on a feature point (point R) of heartbeat signal in an electrocardiogram data fragment of a fixed length, and a sequence of bounding box containing recognition information of point R is output. In the embodiments of the invention, after a recognition output result is obtained, absolute value conversion and non-maximum suppression are further performed on the sequence of bounding box to obtain an optimized sequence of; then, heartbeat classification parameter validity marking is performed in the optimized bounding box; and finally, position information of feature point (point R) of heartbeat signal and valid heartbeat classification information are extracted from the marked sequence for bounding box to generate a sequence of point R position and heartbeat data classification information. By adoption of the method of the invention, the point R in heartbeat signals is used as the feature point of heartbeat signal, so that heartbeat signal data can be reserved to the maximum extent, and various output configuration of analysis report can be made based on the sequence for point R position and heartbeat data classification information.

Those skilled should further appreciate that unit and arithmetic steps described in the embodiments disclosed in this specification may be implemented by electronic hardware, computer software or a combination of these two. To clearly explain the interchangeability of hardware and software, the composition and steps of the illustrative embodiments have been generally described according to functions. Whether these functions are implemented by hardware or software depends on specific applications and design constraints of the technical solution. For each specific application, those skilled in the art may fulfill the functions described through different methods, which should not be construed as being out of the scope of the invention.

The steps of the method or algorithm described in the embodiments disclosed in this specification may be implemented by hardware, software modules executed by a processor, or a combination of these two. The software module may be configured in a random-access memory (RAM), an internal memory, a read-only memory (ROM), an electrically programmable ROM, an electrically erasable and programmable ROM, a register, a hard disk, a removable disk, a CD-ROM, or a storage medium in any other forms in the art.

The purposes, technical solutions and beneficial effects of the invention are explained in further detail with reference to the above specific implementations. It can be understood that the above implementations are merely specific ones of the invention, and are not used to limit the protection scope of the invention. Any amendments or equivalent substitutions and improvements made based on the spirit and principle of the invention should also fall within the protection scope of the invention.

## Claims

1. A heartbeat data classification method based on a point R, comprising:
obtaining one-dimensional electrocardiogram data of which a time length is a preset fragment time threshold to generate an electrocardiogram data fragment, and calling a target detection algorithm to perform recognition of heartbeat signal data feature on the electrocardiogram data fragment to generate a sequence of first bounding box, wherein the sequence of first bounding box comprises a plurality of first bounding boxes;
performing absolute value conversion on all the first bounding boxes in the sequence of first bounding box to generate a sequence of second bounding box, and performing non-maximum suppression on the sequence of second bounding box, wherein the sequence of second bounding box comprises a plurality of second bounding boxes, each said second bounding box comprises a group of point R heartbeat classification probability, and the group of heartbeat classification probability comprises at least one class of parameters for heartbeat classification probability ;
performing valid parameter and invalid parameter marking on all parameters of the heartbeat classification probability in the group of heartbeat classification probability of all the second bounding boxes in the sequence of second bounding box; and
performing point R position information and valid parameter extraction on all the second bounding boxes in the sequence of second bounding box in chronological order to generate a sequence for point R position and heartbeat data classification information.

2. The heartbeat data classification method based on a point R according to Claim 1, wherein the step of obtaining one-dimensional electrocardiogram data of which a time length is a preset fragment time threshold to generate an electrocardiogram data fragment and calling a target detection algorithm to perform recognition of heartbeat signal data feature on the electrocardiogram data fragment to generate a sequence of first bounding box, comprises:
obtaining the one-dimensional electrocardiogram data of which the time length is the preset fragment time threshold to generate the electrocardiogram data fragment; and
calling the target detection algorithm to perform average grid division on the electrocardiogram data fragment with a preset grid time threshold as a grid division step to generate a fragment grid group, performing recognition of heartbeat signal data feature on fragment grids to generate the plurality of first bounding boxes, and collecting all the first bounding boxes generated by all the fragment grids in chronological order of the grids to generate the sequence of first bounding box, wherein the fragment grid group comprises a plurality of fragment grids, each said first bounding box comprises a first heartbeat signal probability, point R relative time data, a QRS normalized time width and a group of first heartbeat classification probability, and the sequence of first bounding box comprises the plurality of first bounding boxes.

3. The heartbeat data classification method based on a point R according to Claim 2, wherein the step of performing absolute value conversion on all the first bounding boxes in the sequence of first bounding box to generate a sequence of second bounding box and performing non-maximum suppression on the sequence of second bounding box comprises:
Step 31: initializing the sequence of second bounding box to be null; initializing the value of a first index to 1, and initializing a first total number to the total number of the first bounding boxes in the sequence of first bounding box;
Step 32: setting a second bounding box corresponding to the first index; initializing a second heartbeat signal probability of the second bounding box corresponding to the first index to be null, initializing point R absolute time data of the second bounding box corresponding to the first index to be null, initializing a QRS absolute time width of the second bounding box corresponding to the first index to be null, and initializing the group of heartbeat classification probability for the second bounding box corresponding to the first index to be null;
Step 33, setting the probability of second heartbeat signal of the second bounding box corresponding to the first index to the probability of first heartbeat signal of the first bounding box, corresponding to the first index, in the sequence of first bounding box, and setting the group of heartbeat classification probability of the second bounding box corresponding to the first index to the group of first heartbeat classification probability of the first bounding box, corresponding to the first index, in the sequence of first bounding box;
Step 34, extracting the point R relative time data of the first bounding box, corresponding to the first index, in the sequence of first bounding box to generate an intra-grid time migration data, adding 1 to a result obtained by rounding a quotient of a difference, obtained by subtracting 1 from the first index, and a preset threshold of the number of bounding boxes in unit grids to generate a grid index of the bounding box, generating grid starting time data according to a product of a difference, obtained by subtracting 1 from the grid index of the bounding box, and the preset grid time threshold, and setting the point R absolute time data of the second bounding box corresponding to the first index to the sum of the grid starting time data and the intra-grid time migration data;
Step 35, extracting the QRS normalized time width of the first bounding box, corresponding to the first index, in the sequence of first bounding box to generate a time width normalized value, and setting the QRS absolute time width of the second bounding box, corresponding to the first index to a product of a square of the time width normalized value and the preset fragment time threshold;
Step 36, adding the second bounding box corresponding to the first index into the sequence of second bounding box as a bounding box object;
Step 37, adding 1 to the value of the first index;
Step 38, determining whether the first index is greater than the first total number; if the first index is greater than the first total number, performing Step 39; or, if the first index is smaller than or equal to the first total number, performing Step 32;
Step 39, performing query of heartbeat signal probability sequential all the second bounding boxes in the sequence of second bounding box; and when the probability of second heartbeat signal of a currently queried second bounding box is out of a preset threshold range of heartbeat probability, deleting the currently queried second bounding box from the sequence of second bounding box; and
Step 40, comparing every two of all the second bounding boxes in the sequence of second bounding box; when a time overlap proportion of the two compared second bounding boxes is out of a preset bounding box overlap proportion threshold range, deleting the second bounding box with a probability of smaller second heartbeat signal in the two compared second bounding boxes from the sequence of second bounding box.

4. The heartbeat data classification method based on a point R according to Claim 1, wherein the step of performing valid parameter and invalid parameter marking on all parameters of the heartbeat classification probability in the group of heartbeat classification probability of all the second bounding boxes in the sequence for second bounding box comprises:
sequentially querying all the second bounding boxes in the sequence of second bounding box, making a maximum parameter of heartbeat classification probability in the group of heartbeat classification probability of a currently queried second bounding box as a valid parameter, and marking the other parameters of heartbeat classification probability smaller than the maximum parameter of heart heartbeat classification probability in the group of heartbeat classification probability of the currently queried second bounding box as invalid parameters.

5. The heartbeat data classification method based on a point R according to Claim 3, wherein the step of performing point R position information and valid parameter extraction on all the second bounding boxes in the sequence of second bounding box in chronological order to generate a sequence for point R position and heartbeat data classification information comprises:
Step 51, chronologically reordering all the second bounding boxes in the sequence of second bounding box according to the point R absolute time data;
Step 52, initializing the sequence of point R position and heartbeat data classification information to be null; initializing a first temporary sequence to be null; initializing the value of a second index to 1, and initializing a second total number to the total number of the second bounding boxes in the sequence of second bounding box;
Step 53, setting information for second index point R position and heartbeat data classification; initializing point R position information of the second index point R position and heartbeat data classification information to be null, and initializing QRS width information of the second index point R position and heartbeat data classification information to be null; initializing a group of valid heartbeat classification probability of the second index point R position and heartbeat data classification information to be null;
Step 54, calculating the total number of parameters of heartbeat classification probability, marked as valid parameters, in the group of heartbeat classification probability of the second bounding box, corresponding to the second index, in the sequence of second bounding box to generate the total number of valid classification parameters;
Step 55, determining whether the total number of valid parameters of classification is equal to 0; if the total number of valid parameters of classification is greater than 0, performing Step 56; or, if the total number of valid parameters of classification is equal to 0, performing Step 58;
Step 56, setting the point R position information of the point R position and heartbeat data classification information with the second index to the point R absolute time of the second bounding box, corresponding to the second index, in the sequence of second bounding box; setting the QRS width information of the point R position and heartbeat data classification information with the second index to the QRS absolute time width of the second bounding box, corresponding to the second index, in the sequence of second bounding box; extracting all parameters of probability for heartbeat classification , marked as valid parameters, in the group of heartbeat classification probability of the second bounding box, corresponding to the second index, in the sequence of second bounding box, and sequentially adding parameters of heartbeat classification probability into the group of valid heartbeat classification probability of the point R position and heartbeat data classification information with the second index;
Step 57, adding the point R position and heartbeat data classification information with the second index into the first temporary sequence;
Step 58, adding 1 to the value of the second index;
Step 59, determining whether the second index is greater than the second total number; if the second index is greater than the second total number, performing Step 60; or, if the second index is smaller than or equal to the second total number, performing Step 53; and
Step 60, extracting all point R position and heartbeat data classification information in the temporary sequence, and sequentially adding the point R position and heartbeat classification information into the sequence for point R position and heartbeat data classification information.

6. A device, comprising a memory and a processor, wherein the memory is used to stores a program, and the processor is used to implement the method according to anyone of Claims 1-5.

7. A computer program product including an instruction, enabling a computer to implement the method according to any one of Claims 1-5 when running on a computer.

8. A computer-readable storage medium, comprising an instruction, wherein when the instruction runs on a computer, the computer is enabled to implement the method according to any one of Claims 1-5.
